# EUROPEAN PATENT SPECIFICATION

(11) **EP 1 005 353 B1**
(45) Date of publication and mention of the grant of the patent: **14.09.2005**
(21) Application number: 98928734.7
(22) Date of filing: 02.06.1998
(51) Int. Cl.: A61K 35/74, C12N 1/20, A61P 1/12

(54) **PHARMACEUTICAL PREPARATION COMPRISING LACTOBACILLUS CASEI RHAMNOSUS**
LACTOBACILLUS CASEI RHAMNOSUS-HALTIGE PHARMAZEUTISCHE FORMULIERUNG
PREPARATION PHARMACEUTIQUE CONTENANT DU LACTOBACILLUS CASEI RHAMNOSUS

(30) Priority: 02.06.1997 SE 9702083
(43) Date of publication of application: 07.06.2000
(73) Proprietor: Essum AB, 907 40 Umea (SE)
(72) Inventor: GRAHN HAKANSSON, Eva, S-903 31 Umea (SE); HAKANSSON, Stellan, S-903 31 Umea (SE)
(74) Representative: Wiklund, Erik
(86) International application number: PCT/SE1998/001041
(87) International publication number: WO 1998/055131

(56) References cited:
- EP-A- 0 271 364
- WO-A-93/01823
- DE-A- 2 421 066
- DIALOG INFORMATION SERVICES, File 65, Inside Conferences, Dialog Accession No. 02105717, Inside Conferences Accession No. CN022056031, HAAKANSSON E.G. et al., "The Ability of Lactobacillus Casei Rhamnosus LB21 to Survive in the Gastrointestinal Tract and to Prevent Antibiotic Associated Diarrhoea"; & VTT SYMPOSIUM, 1997, Vol. 173, p. 57-58.

## Description

The present invention relates to a pharmaceutical preparation for prophylaxis against and/or treatment of gastrointestinal disorders in man and animals.

### Background

A health problem which is widely spread in many contexts is diarrhoea and other enteric disorders caused by various pathogenic microorganisms. The problems are particularly common in people that have been treated with antibiotics or radiation in cancer treatment, in people suffering from stress, in tourists travelling abroad, and in day-care centres. Examples of intestinally pathogenic bacteria which thus cause diarrhoea are Salmonella, Shigella, Yersinia, E.coli, Pseudomonas, Clostridium difficile and sordelli, Stafylococcus aureus, Klebsiella, Helicobacter pyroli and Campylobacter.

It has been widely known for a long time that microorganisms affect each other positively or negatively by promoting or inhibiting each other's growth. This interference phenomenon has been studied above all in the skin flora, the pharynx and the intestines of man. The underlying mechanisms are not fully known, but it has been found that the normal bacterial flora is very important in the defence against pathogenic bacteria. Invading bacteria are inhibited by the bacteria in the normal flora in different ways, one of which is the production of antibiotic-like substances, so-called bacteriocins. In contrast to antibiotics, the bacteriocins have in most cases a very selective effect on a group of bacteria, but do not affect the remaining bacterial flora. The possibility of administering harmless bacteria with such a specific effect to patients suffering from infection has not only been discussed but has also been made use of to some extent. It goes without saying that the administered bacteria must be able to establish themselves in the environment that is to be affected. For instance, this has been the idea of giving sour milk to patients having a disturbed intestinal flora, caused by severe diarrhoea after being treated with antibiotics. However, it has not been scientifically analysed what has happened in the intestinal flora, and the effect, if any, has in most cases been uncertain. This has probably been due to the fact that no colonisation has taken place in the intestines, or that the added bacteria had no possibility of interfering with the bacteria causing diarrhoea. Experiments have also been made to replace the bacterial flora with harmless microorganisms in the nasal mucous membrane, the skin and the pharynx, and these experiments have proved that a so-called recolonisation is possible and may give the intended effects.

As mentioned above, the experiments that have been made to administer bacteria to patients suffering from various disorders, caused by pathogenic microorganisms, have in most cases given inconclusive results. Apparently the knowledge of which bacterial strains are best suited to achieve colonisation and interference has not been sufficient.

By working under defined conditions, it has in recent years become possible to establish important basic principles of mechanisms which control the interaction between microorganisms and also to develop methods for studying this interaction. This has opened new possibilities of interfering with this interaction in a meaningful, reproducible manner and, consequently, also to therapeutically-prophylactically utilise this in the treatment of infections.

In newborn babies, it is Lactobacillus strains which together with Bifido bacteria first colonise the sterile intestines. Also in adults, Lactobacillus is a dominating normal flora in the intestines. Many Lactobacillus strains in fact produce lactic acid, acetic acid and hydrogen peroxide, which has an inhibitory effect on potentially intestinally pathogenic bacteria. Some of them have effect against specific strains, whereas others have a wider inhibitory effect.

The significance of Lactobacillus strains in the intestinal flora has been demonstrated in many different studies (see the basic literature references 1-8). For instance, it has been shown that certain Lactobacillus strains protect against colonisation of pathogenic bacteria, produce bacteriocins and other antimicrobial components, stimulate the immune system, decrease the risk of tumours and colon cancer, reduce the serum cholesterol content, improve the gastrointestinal mobility of old people, improve the tolerance of lactose etc. There are also studies where it has not been possible to show these effects, depending on the fact that all Lactobacillus strains do not function in the tested respect. In some studies, use has been made of an unsuitable Lactobacillus preparation, e.g. freeze-dried in pulverulent state or as uncoated capsules.

WO 96/38159 (Lafor Lab Ltd) discloses a pharmaceutical composition for treatment of antibiotic-associated diarrhoea, said composition containing, inter alia, viable lactobacillus rhamnosus strains.

Japanese Patent Application 172,949 by Jakult Honsa KK discloses, among other things, lactobacillus rhamnosus and casei as an active ingredient in an anti-allergic agent.

WO 93/01823 concerns a process for isolating of a strain of lactobacillus having the ability of colonising in human intestines. For example, the strain of L. casei sp. rhamnosus 271 has been isolated and is said to be useful for prophylaxis or treatment of bacterial infections, especially in the form of a fermented nutrient composition. The strain at issue has been isolated from the intestines in adults.

EP-A-0 271 364 relates to a preparation for treatment or prophylaxis of enteric disorders in animals. This preparation comprises one or more lactic acid bacteria selected from the phenotypes of, inter alia, Lactobacillus casei spp. rhamnosus. The preparation is a non-specific mixture of a plurality of strains and besides is not intended for use in man. The strains at issue seem to have been isolated from cattle and sheep and are thus not applicable to man. Moreover, experiments have only been made on cattle and sheep.

DT-A1-2,421,066 relates to a preparation for reestablishing a disturbed intestinal flora to a normal state, the preparation comprising a mixture of bifido bacteria and possibly also a Lactobacillus casei var. rhamnosus strain.

In our analyses of the interaction between different bacteria, we have studied, inter alia, how certain Lactobacillus strains can counteract diarrhoea which has occurred owing to the intake of antibiotics and prevent the growth of pathogenic bacteria such as Helicobacter. On a laboratory scale, cultures of Lactobacillus have been tested, from which a great number of clones with varying biological properties have been isolated. One of these, Lactobacillus casei rhamnosus LB21, below also referred to as "LB21", has been isolated from faeces of infants and has proved to have a particularly pronounced inhibitory effect on the majority of the bacteria that are important to different types of infections in man, such as Staphylococcus aureus, different species of Salmonella, Shigella, Pseudomas, Klebsiella-Enterobacter, Campylobactus, Clostridium difficile, Helicobacter pylori etc. The strongly inhibitory effect of precisely LB21 on intestinally pathogenic bacteria is probably due to the fact that it produces a specific low-molecular protein or a specific low-molecular peptide, which is thermally stable, has a molecular weight below 5000 Daltons and which in a unique fashion has a detrimental effect on intestinally pathogenic bacteria.

However, none of the above-mentioned publications describes the specific strain Lactobacillus casei rhamnosus LB21, which has been isolated from faeces of infants and which has a considerably improved effect on gastrointestinal disorders compared with closely related strains.

### Object of the Invention

The object of the present invention is to alleviate the above-mentioned health problems more effectively than allowed by prior-art methods of treatment. This object is achieved by a pharmaceutical preparation of the type mentioned by way of introduction, which in addition has the features defined in the characterising clause of appended claim 1. Preferred embodiments are stated in the appended subclaims.

### Description of the Invention

The present invention relates to a pharmaceutical preparation for prophylaxis against and/or treatment of gastrointestinal disorders in man and animals, said preparation being characterised in that is comprises a viable microorganism strain in the form of the Lactobacillus casei rhamnosus strain LB21, in at least one pharmaceutically acceptable carrier medium, in which the microorganism retains its viability. More specifically, the present invention relates to a pharmaceutical preparation for prophylaxis against and/or treatment of, inter alia, antibiotic-associated diarrhoea, helicobacter infection in the stomach, tourist diarrhoea and stress-induced gastrointestinal disorders.

Moreover, the present invention concerns the Lactobacillus casei rhamnosus strain NCIMB 40564, which is capable of preventing and/or curing gastrointestinal disorders in man and animals, and which has been isolated from faeces of an approx. 1-8-day-old infant.

The inventive pharmaceutical preparation contains the microorganism strain Lactobacillus casei rhamnosus LB21 in an amount of 10²-10¹², preferably 10⁶-10¹⁰, colony-forming units per ml of ready-to-use preparation.

By the expression colony-forming units is meant the number of colonies containing one or more bacteria growing on a substrate.

By the expression helicobacter infection is meant symptoms of gastric ulcer where Helicobacter has been identified to be present in the stomach or the upper part of the small intestine.

By the expression tourist diarrhoea is meant diarrhoea occurring as the gastrointestinal flora changes, for instance, abroad, and also in case of infection caused by e.g. Salmonella and E.coli.

By the expression stress-induced gastrointestinal disorders is meant imbalance in the gastrointestinal flora causing stomach-ache, diarrhoea, gas formation etc.

In a preferred embodiment of the invention, the carrier of LB21 is a soured or fermented milk product, such as soured milk, yoghurt, milk, or it is fruit juice, ice-cream, soup or fruit drinks. The strain of bacteria can be used as starting culture in the preparation of such milk products, resulting in soured or fermented milk products having an increased amount of the advantageous strain. Of course it is also possible to add LB21 to prepared milk products or to fruit juice, soup or ice-cream to obtain in this manner a greater amount of desirable bacteria in the medium. This embodiment is highly important since the thus obtained preparation is a wholesome and appreciated foodstuff. Such a preparation can be given prophylactically or curatively against diarrhoea and other intestinal infections that frequently affect infants. Besides it can be given to people treated with antibiotics or radiation in cancer treatment, to people having a helicobacter infection in the stomach and to tourists travelling abroad, where a bacterial flora that is unfamiliar to the gastrointestinal duct is frequently encountered.

The preparation can also be present in dry form for oral administration, for instance, in the form of capsules, tablets or powder or in the form of a suspension, optionally frozen, such as NaCl solution, glucose solution or skim milk. The carrier media here used are of a conventional type and well known to those skilled in the art. The capsules can be of a type resistant to gastric juice, such that the bacteria are protected against the gastric juice and only released in the intestines. For the same reason, tablets can be administered with a prior-art type of coating resistant to gastric juice. Powders can be intended to be stirred into an aqueous liquid before administration, for instance, water, fruit juice or some milk product.

The preparation has thus been found to have a particularly great potential as a prophylactic agent against and/or an agent for treating antibiotic-associated diarrhoea.

The preparation can also advantageously be used for prophylaxis against and/or treatment of helicobacter infection in the stomach and the upper small intestine in man and animals, but also against so-called tourist diarrhoea and stress-induced gastric disorders.

In the pharmaceutical preparation according to the present invention, some other lactic acid bacterium can possibly also be incorporated, such as one or more Lactococcus or Lactobacillus strains, but preferably the strain of Lactococcus lactis L1A is also incorporated.

The pharmaceutical preparation according to the present invention has also been found to be particularly advantageous in prophylaxis and/or treatment of children in the following situations.
a) Newborn babies and prematurely born babies in need of intensive care.
   Such intensive care can result in the fact that the normal colonisation of the intestines with Lactobacilli and bifido bacteria does not take place, which increases the risk of pathogenic bacteria dominating. This also increases the risk of infections and use of antibiotics. The adding of LB21 can normalise the intestinal flora in these babies and counteract the unfavourable development as described above.
b) Babies exposed to an increased risk of getting type 1 diabetes, celiaki (gluten allergy) and various autoimmune conditions.
   A factor causing these disorders probably is disturbed immune control in the intestines such that antigens (substances not occurring naturally in the body) from food, intestinal bacteria, virus or other microorganisms may cause an immune reaction against the own tissues of the body. Adding LB21 can normalise the intestinal flora and, thus, counteract the occurrence of these disorders.
c) Children having undergone an operation for bile duct atresia (congenital malformation with complete or partial loss of bile ducts in and/or outside the liver) and children having undergone a liver transplant.

These categories of children run an increased risk of being stricken with infections affecting liver/bile ducts after the operation. The supplying of LB21 can reduce the risk of such complications by inhibiting the growth of the bacteria that usually cause these types of infections.

The inventive pharmaceutical preparation is also useful in connection with cytotoxic treatment. Patients with cancer diseases are frequently treated with cytotoxins which have a negative effect on the function of the intestines and have a detrimental effect on the structure and new growth of the intestinal mucous membrane. In consequence, this type of treatment often causes a reduced assimilation of nutrients and diarrhoea. A further consequence is that intestinal bacteria can spread to the blood vessels through the weakened intestinal mucous membrane and cause serious infections. This leads to an increased consumption of antibiotics, which in turn further deteriorates the function of the intestines and may imply that various intestinal bacteria develop resistance to antibiotics.

By supplying LB21 in connection with the treatment with cytotoxins, the negative effects on the function and structure of the intestinal mucous membrane can be reduced, thereby reducing the risk of serious infections caused by bacteria in the intestines.

Moreover, the pharmaceutical preparation according to the present invention is useful for preventing allergy in children, e.g. atopic eczema, and for preventing and treating constipation, above all in old people.

When the pharmaceutical preparation according to the invention is intended for treatment of animals, the carrier is a suitable feedstuff, such as in the form of whey, dry fodder or a concentrated suspension.

The microorganism should be stored in a freeze-dried state, preferably in skim milk in a dark and dry place, but also frozen at a temperature of about -70°C.

The necessary dose of the inventive preparation can be adjusted in dependence on such factors as the patient's age and condition, as well as the type and degree of difficulty of the illness. It is within the competence of a person skilled in the art to determine a suitable dose against this background. A further advantage of the invention is a that a great overdose of the pharmaceutical operation hardly means a risk to the patient.

### Inventive microorganism

The above-mentioned microorganism strain Lactobacillus casei rhamnosus LB21 was isolated as one of about hundred Lactobacillus strains from faeces of an approx. 5-day-old healthy baby and was then selected to be the most effective strain for the inventive purpose. It is very important that LB21 be isolated from the baby in the first week, such as from the first to the eighth day of living, preferably approximately on the fifth day of living. LB21 is distinguished from other Lactobacillus casei rhamnosus strains by using pulsated field gel electrophoresis (PFGE) according to the Fasola and BioRad method. LB21 has previously not been isolated and defined and thus is a previously completely unknown Lactobacillus casei rhamnosus strain, which has been found to have particularly favourable effects in the above-mentioned health problems and states of ill-health.

The following Table shows test results from the initial stage of selection, where LB21 is compared with other Lactobacillus strains which have been isolated from faeces of babies in respect of the inhibitory effect on several pathogens.

| Interference Table | | | | | | | |
|---|---|---|---|---|---|---|---|
| Pathogen | LB 6 | LB 8 | LB 14 | LB 18 | LB 21 | LB 27 | LB 32 |
| E.coli | 4/5 | 1/5 | 0/5 | 2/5 | 5/5 | 0/5 | 1/5 |
| Klebsiella | 2/5 | 2/5 | 1/5 | 2/5 | 5/5 | 1/5 | 3/5 |
| Ent.cocc | 0/5 | 0/5 | 0/5 | 1/5 | 3/5 | 0/5 | 1/5 |
| GBS | 2/5 | 0/5 | 0/5 | 2/5 | 5/5 | 0/5 | 2/5 |

As mentioned above, the LB21 strain is included as an active ingredient in the inventive preparation and was deposited on 11 June 1993 at The National Collection of Industrial and Marine Bacteria (NCIMB), 23 St. Machar Drive, Aberdeen AB2 1RY, Great Britain. Its accession number is NCIMB 40564. It should be noted that the strain was deposited with the identification reference Lactobacillus casei rhamnosus L20 05, but that the usual designation of the strain has later been changed to Lactobacillus casei rhamnosus LB21. The strain is typified or classified according to the API 50CH-system (API-System - La Balme les Grottes - 38390 Montalieu - Vercieu), which is apparent from the following Typifying Table.

### API 50 CL

37°C, CO₂ blood agar

| | Compound | Code | 24 h | 48 h |
|---|---|---|---|---|
| 0 | Check | - | - | - |
| 1 | Glycerol | - | - | - |
| 2 | Erythritol | - | - | - |
| 3 | D-arabinose | - | - | - |
| 4 | L-arabinose | - | - | - |
| 5 | Ribose | + | + | + |
| 6 | D-xylose | - | - | - |
| 7 | L-xylose | - | - | - |
| 8 | Adonitol | - | - | - |
| 9 | β-methylxyloside | - | - | - |
| 10 | Galactose | + | + | + |
| 11 | D-glucose | + | + | + |
| 12 | D-fructose | + | + | + |
| 13 | D-mannose | + | + | + |
| 14 | L-sorbose | - | - | - |
| 15 | Rhamnose | G | G | G |
| 16 | Dulcitol | + | G | + |
| 17 | Inositol | - | - | - |
| 18 | Mannitol | + | + | + |
| 19 | Sorbitol | + | G | + |
| 20 | α-methyl-D-mannoside | - | - | - |
| 21 | α-methyl-D-glucoside | - | - | - |
| 22 | N-acetylglucoseamine | + | + | + |
| 23 | Amygdalin | G | - | G |
| 24 | Arbutin | + | - | + |
| 25 | Esculin | + | + | + |
| 26 | Salicin | + | + | + |
| 27 | Cellobiose | + | - | + |
| 28 | Maltose | - | - | - |
| 29 | Lactose | + | + | + |
| 30 | Melibiose | - | - | - |
| 31 | Saccharose | - | - | - |
| 32 | Trehalose | + | + | + |
| 33 | Inulin | - | - | - |
| 34 | Melezitose | + | - | + |
| 35 | D-raffinose | - | - | - |
| 36 | Amidon | - | - | - |
| 37 | Glycogen | - | - | - |
| 38 | Xylitol | - | - | - |
| 39 | β-gentiobiose | G | - | G |
| 40 | D-turanose | - | - | - |
| 41 | D-lyxose | - | - | - |
| 42 | D-tagatose | + | + | + |
| 43 | D-fucose | - | - | - |
| 44 | L-fucose | - | - | - |
| 45 | D-arabitol | - | - | - |
| 46 | L-arabitol | - | - | - |
| 47 | Gluconate | G | G | G |
| 48 | 2-cetogluconate | - | - | - |
| 49 | 5-cetogluconate | - | - | - |
| G = green colour | | | | |
| + = complete change to yellow colour | | | | |
| - = original blue-lilac colour | | | | |

It is here important to emphasise that there is a great difference between different Lactobacillus strains, as is also the case in the subspecies Lactobacillus casei rhamnosus (LB21). LB21 in the pharmaceutical preparation according to the present invention has certainly been identified as a Lactobacillus casei rhamnosus strain, but this is far from meaning that the strain at issue is identical with or similar to other Lactobacillus casei rhamnosus strains described in the literature, such as in WO 93/01823. These subspecies grow, for instance, differently well in oxygen environment and at different temperatures. It is evident that it is not a matter of identical strains, and a detailed characterisation of the respective subspecies (cf. above) shows that they have different properties, which a person skilled in the art realises. A great difference is, for instance, that LB21 has been isolated from faeces of a baby, whereas the strain 271 in WO 93/01823 has been isolated from the intestines of adults.

### Performed Experiments

### Passage through the Gastrointestinal Duct

A requirement for probiotic strains of lactic acid bacteria, such as LB21 in the inventive preparation, to be able to act in the small and thick intestine of man and animals is that they must survive the passage through the acid environment of the stomach. A study has been made to prove that Lactobacillus casei rhamnosus LB21 could survive the passage through the stomach and be found in the faeces of healthy volunteers. Moreover, we have studied for how long this strain could be found in the faeces after the subjects of the experiment had discontinued the oral intake of the same.

In a part experiment A, 25 healthy voluntary people supplied a sample of faeces in a sterile plastic tube. The sample was stored in a cold place (4°C max) up to the occasion of cultivation. Before cultivation, the sample was mixed into a 0.9% salt solution at a ratio of 1:10. Then 10 µl of the sample was cultured on Rogosa plates with 128 µ/ml vancomycin. The plates were incubated at 37°C in CO₂ environment and were read after 48 h. The limit for participating in part experiment B was set to be at most 4x10⁴ colony-forming units of vancomycin-resistant lactobacilli per g of faeces.

In part experiment B, 13 of the 25 above-mentioned people participated, having a relatively vancomycin-sensitive lactobacilli flora in the intestines. The objects were instructed not to eat foodstuff containing lactobacilli from 2 weeks before the start of the study including the last day of the study. Each object had in connection with a meal 10 ml salt-solution with 10¹¹ colony-forming units of viable LB21 a day for 5 days. These objects supplied a sample of faeces the day before the intake of lactobacilli (zero sample) and then from the 4th day of the LB21 intake up to and including 12 days after the completed cure. Sampling and cultivation of samples were carried out according to the same method as in part experiment A. The identification of Lactobacillus casei rhamnosus LB21 was carried out by means of gram colouring and API classification (CHL-media).

It should be noted that object No. 8 took penicillin in the course of the study. This object therefore took LB21 for 10 days starting at the same time as the rest of the group of objects.

Part experiment B was interrupted 12 days after the intake of LB21 had ceased. This took place although LB21 could still be found in the faeces of 4 of 13 objects. In the remaining 9 objects, LB21 could be isolated from the faeces on average 5 days after the intake had ceased. In object No. 8 taking penicillin, LB21 was found in the faeces during the entire penicillin cure and 2 days after the intake had ceased. In the samples of faeces, LB21 was found in the order of 1x10³ to 5x10⁵ colony-forming units per g of faeces.

| Object | Number of days after the cessation of the treatment LB21 present in faeces |
|---|---|
| 1 | 3 |
| 2 | 6 |
| 3 | 3 |
| 4 | 12 |
| 5 | 12 |
| 6 | 3 |
| 7 | 5 |
| 8 | 2 |
| 9 | 7 |
| 10 | 12 |
| 11 | 3 |
| 12 | 7 |
| 13 | 12 |

Summing up, it could be established that the Lactobacillus casei rhamnosus strain LB21 survived the passage through the stomach and could be found in the faeces of all objects, also the object who had undergone a penicillin treatment. 4 of the 13 objects still had LB21 in the faeces 12 days after the cessation of oral intake of LB21. The remaining 9 objects still had LB21 on average 5 days after the intake of LB21 had ceased. The average number for the entire group of 13 objects was 6 days.

### Inhibitory Effect of Lactobacillus casei rhamnosus LB21

The above-mentioned lactic acid bacterium LB21 has been tested for its inhibitory effect on a large number of intestinally pathogenic bacteria. It has been found to have a satisfactory inhibitory effect, and an experiment was also carried out for the purpose of investigating if it could also inhibit E.coli 0-157, Clostridium difficile and Helicobacter pylori.

Two strains of E.coli 0-157, one being tox- and the other being tox+, were isolated from patients. Five strains of Clostridium difficile from patients suffering from antibiotic-associated diarrhoea, and three strains of Helicobacter pylori from clinical material (biopsies) were also tested. In the performed interference test use was made of the agar layer method, which was adjusted to the bacteria tested. This method means that the Lactobacillus strains are cultivated in MRS broth (Merck) at 37°C in 5% CO₂ overnight. Each strain was then moulded separately into MRS agar (25 ml agar). The agar plates were incubated in the above-mentioned conditions for 24 h. A new agar layer (M17 agar(Merck), 25 ml) or blood agar (BHI + horse blood) was poured over the first layer, and the second layer is then allowed to solidify for 4 h. The test bacteria were then cultivated separately in TY medium (Holm, S E and Falsen, E, APMIS 1967; 69, 264) at 37°C. The bacteria were passed to Bertani troughs (0.25 ml, 10⁶ bacteria per ml). The bacteria were diluted to 1:10 in NaCl in a new Bertani trough. From these troughs the test strains (25 per trough) were then stamped on the agar plate by means of a Steers steel pin replicator. The plates were incubated at 37°C for E.coli, at 37°C and anaerobically for Clostridium difficile and at 37°C in microaerophilic environment for Helicobacter pylori. (Steers E et al, J. Antibiot. Chemoter. 179; 9, 307). For checking, plates without LB were used.

The LB21 strain inhibited all the tested bacteria.

The final result showed that the LB21 strain had an excellently satisfactory inhibitory effect on these intestinal pathogens, which is apparent from the following Table.

| Inhibitory Effect of Lactobacillus LB21 | | | |
|---|---|---|---|
| Test bacteria | Number | Number inhibited | % inhibition |
| E.coli | 48 | 48 | 100 |
| Enterococci | 41 | 34 | 83 |
| Group B-streptococci | 100 | 100 | 100 |
| Group A-streptococci | 5 | 5 | 100 |
| Klebsiella | 36 | 36 | 100 |
| Proteus mir. | 16 | 16 | 100 |
| Salmonella | 15 | 15 | 100 |
| Shigella | 15 | 15 | 100 |
| Ps. aureginosa | 15 | 15 | 100 |
| Yersinia | 10 | 10 | 199 |
| Staphylococcus sapro | 6 | 4 | 67 |
| Lactobacilli | 11 | 0 | 0 |
| H. pylori | 3 | 3 | 100 |
| E. coli 0-157 | 2 | 2 | 100 |
| Clostridium difficile | 5 | 5 | 100 |

The tests were carried out according to the agar layer method as well as in broth. They were repeated and carried out at several different pH values.

### Capability of Lactobacillus casei rhamnosus LB21 of inhibiting antibiotic-associated diarrhoea

31 healthy objects participated in the study, 16 men and 15 women. The objects were then divided according to sex and then randomly into test or control group. The test group included 16 objects and the control group 15 objects. The average age in the test group was 24.6 years, in the range 19-31 years. The average age in the control group was 24.4 years, in the range 20-33 years. Two objects in the control group dropped out in the course of the study owing to pregnancy and trouble caused by the intake of antibiotics. The dropping out thus amounted to 6.5%. For the last faeces test, one more object in the control group dropped out owing to a sports injury. None of the objects was smoker or snuff taker or had taken antibiotics during the last six months before the study. No medication except Kåvepenin was allowed in the course of the study. The objects were not allowed to take any fermented products two weeks before the start of the intake of penicillin and in the course of the study. The experiment was carried out as a double blind placebo-controlled test and was approved by the search-ethical committee of the Faculty of Medicine, University of Umeå, Umeå.

The test group as well as the control group took 500 mg Kåvepenenin in capsules perorally (phenoxymethyl-penicillin potassium, Astra Läkemedel, Södertälje) twice a day for ten days.

The test group was given frozen skim milk with LB21 (Essum, Umeå) at a concentration of 10¹¹ cfu (colony-forming units) per ml. The control group was given skim milk as placebo. The objects in the test group took 5 ml of frozen skim milk with LB21 or placebo twice a day. The intake started simultaneously with the penicillin and lasted 15 days.

Samples of faeces were collected before the intake of Kåvepinin on day 0, during the intake of Kåvepenin and LB21 or placebo on day 10 and on day 15 during the intake of LB21/placebo after the completed Kåvepenin cure. The samples were stored in a cold place (+4-8°C) before being delivered to the laboratory where they were frozen and stored at -20°C before analysis. All samples were frozen within 24 h of the sampling.

The objects recorded the number of defecations a day and the consistency of the faeces in a form from one week before the intake of Kåvepenin up to and including five days after the completed cure. Stomach ache and other subjective symptoms were also recorded.

The samples of faeces were weighed and diluted with sterile water at a ratio of 1:10. The pH was measured with indicator paper (Merck, pH 5-10) in the middle of the mixture. LB21 was cultivated after being diluted with sterile water on Rogosa plates with 128 µg of vancomycin. The samples were incubated at 37°C in CO₂ environment for 48 h.

All lactobacilli were cultivated after being diluted with sterile water on Rogosa plates. The samples were anaerobically incubated at 37°C for 48 h. Then the samples were stored at -20°C.

Tests for Clostridium difficile toxin were carried out according to Aronsson et al.

The frequency of defecations, consistency of the faeces, stomach ache and other subjective symptoms were recorded, and average, median and range were calculated.

Statistical calculations were carried out with SPSS for Windows. The results were presented as average values and median values. The values of lactobacilli were logarithmated (log 10) before the statistical calculation. The median value and the range between the maximum and minimum value were calculated on the basis of the number of defecations, consistency of the faeces and pH value. Statistical comparisons between the groups, where a normal range was not to be found or was unreliable, were carried out with Mann-Whitney U test (number of defecations, consistency of faeces, lactobacilli, pH). A pH of less than 0.05 was considered statistically significant.

### Results

The objects taking LB21 had considerably fewer defecations/diarrhoea during the intake of phenoxymethyl penicillin compared with the placebo group, viz. 0.24 and 0.98, respectively (p<0.05). Moreover, the frequency of defecations and the occurrence of stomach ache increased to a considerable extent during the penicillin treatment in the placebo group compared with the situation before the intake of the penicillin. There was also a considerable increase of the amount of Lactobacillus casei rhamnosus in faeces in the group that had been supplied with LB21. There were, however, no considerable differences in the frequency of defecations or in respect of the total number of lactobacilli in the samples of faeces in the two groups. Furthermore, there were no cases of Clostridium difficile toxin in any of the two groups.

### Literature References:

1. Sandine, W.E. 1979. Roles of lactobacillus in the gastrointestinal tract. J. Food Protect. 42, 259-262.
2. Fuller, R. 1986. Probiotics. J. Appl. Bacteriol. Symp. Suppl. 1S-7S.
3. Gilliland, S.E., Nelson, C.R. och Maxwell, C. 1985. Assimilation of cholesterol by Lactobacillus acidophilus. Appl. Environ. Microbiol. 33, 15-18.
4. Goldin, B.R. and Gorbach, S.L. 1980. Effect of Lactobacillus acidodophilus dietary supplements on 1,2-dimetylhydrazine dihydrochlorid induced intestinal cancer in rats. J. Natl. Cancer. Inst. 64, 263-271.
5. Goldin, B.R. and Gorbach, S.L. 1987. Lactobacillus GG: a new strain with properties favorable for survival, adhesion and antimicrobial activity in the gastrointestinal tract. FEMS Microbiol. Rev. 46, P72.
6. Lessard, M. and Brisson, G.J. 1987. Effect of lactobacillus fermentation product on growth immune response and fecal enzyme activity in weaned pigs. Can. J. Anim. Sci. 62, 509-516.
7. Siitonen, S., Vapaatalo, H., Salminen, S., Goldin, A., Saxelin, M., Wikberg, R. and Kirkkola, A-L. 1990. Effect of lactobacillus GG Yoghurt in prevention of antibiotic associated diarrhoea. Annual Med. 22, 57-59.
8. Fernandes, C., Shahani, K. Amer, M. 1987. Therapeutic role of dietary lactobacilli and lactobacilli fermented dairy products. FEMS microbiol. Rew. 46: 343-356.

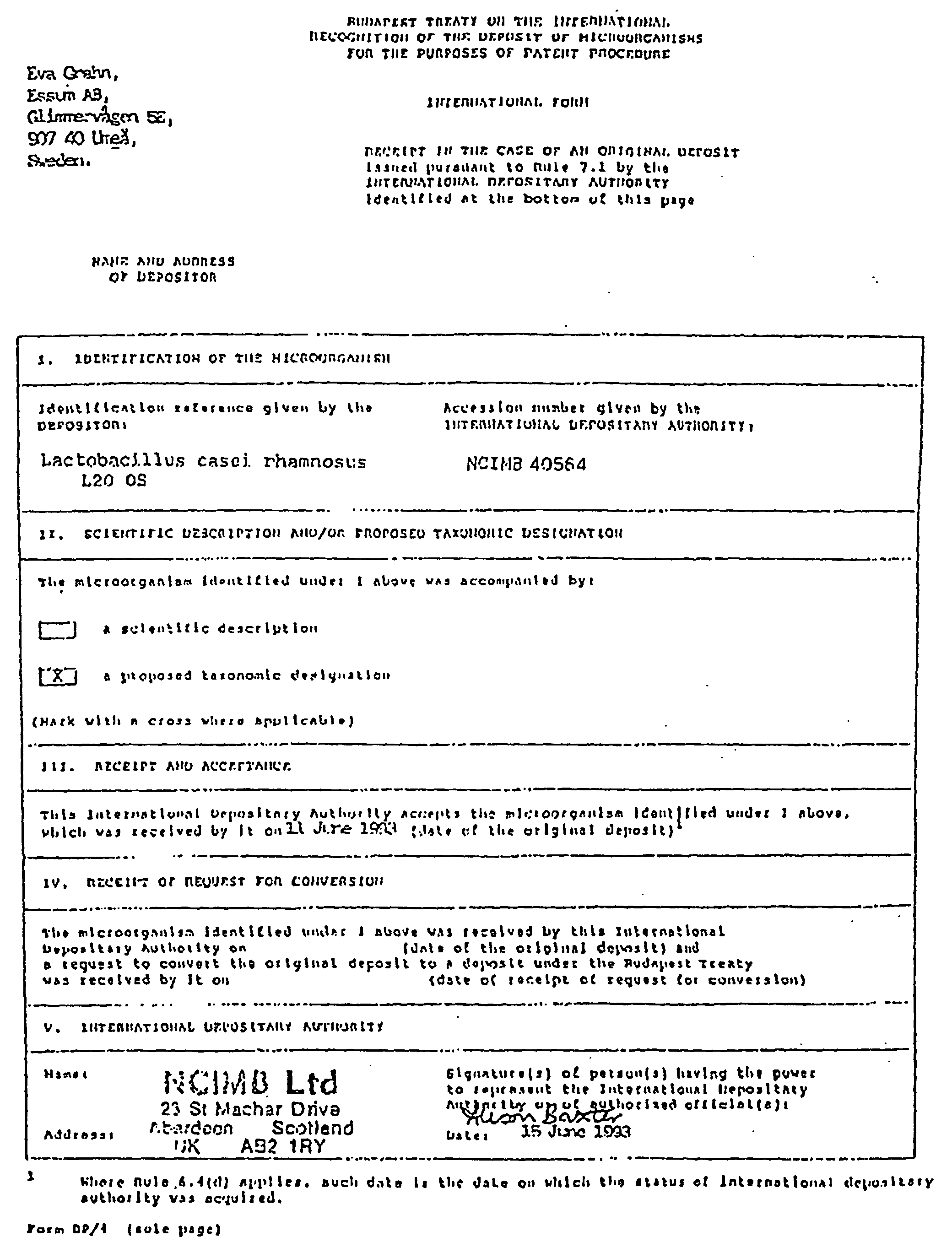
BUDAPEST TREATY ON THE INTERNATIONAL
RECOGNITION OF THE DEPOSIT OF MICROORGANISMS
FOR THE PURPOSES OF PATENT PROCEDURE

Eva Grahn,
Essum AB,
Glimmervägen 5E,
907 40 Umeå,
Sweden.

INTERNATIONAL FORM

RECEIPT IN THE CASE OF AN ORIGINAL DEPOSIT
issued pursuant to Rule 7.1 by the
INTERNATIONAL DEPOSITARY AUTHORITY
identified at the bottom of this page

NAME AND ADDRESS
OF DEPOSITOR

I. IDENTIFICATION OF THE MICROORGANISM

Identification reference given by the DEPOSITOR: | Accession number given by the INTERNATIONAL DEPOSITARY AUTHORITY:

Lactobacillus casei rhamnosus | NCIMB 40564
L20 OS

II. SCIENTIFIC DESCRIPTION AND/OR PROPOSED TAXONOMIC DESIGNATION

The microorganism identified under I above was accompanied by:

[ ] a scientific description

[X] a proposed taxonomic designation

(Mark with a cross where applicable)

III. RECEIPT AND ACCEPTANCE

This International Depositary Authority accepts the microorganism identified under I above,
which was received by it on 11 June 1993 (date of the original deposit)¹

IV. RECEIPT OF REQUEST FOR CONVERSION

The microorganism identified under I above was received by this International
Depositary Authority on (date of the original deposit) and
a request to convert the original deposit to a deposit under the Budapest Treaty
was received by it on (date of receipt of request for conversion)

V. INTERNATIONAL DEPOSITARY AUTHORITY

Name: NCIMB Ltd | Signature(s) of person(s) having the power
23 St Machar Drive | to represent the International Depositary
Address: Aberdeen Scotland | Authority or of authorized official(s):
UK AB2 1RY |
| Date: 15 June 1993

¹ Where Rule 6.4(d) applies, such date is the date on which the status of international depositary
authority was acquired.

Form BP/4 (sole page)

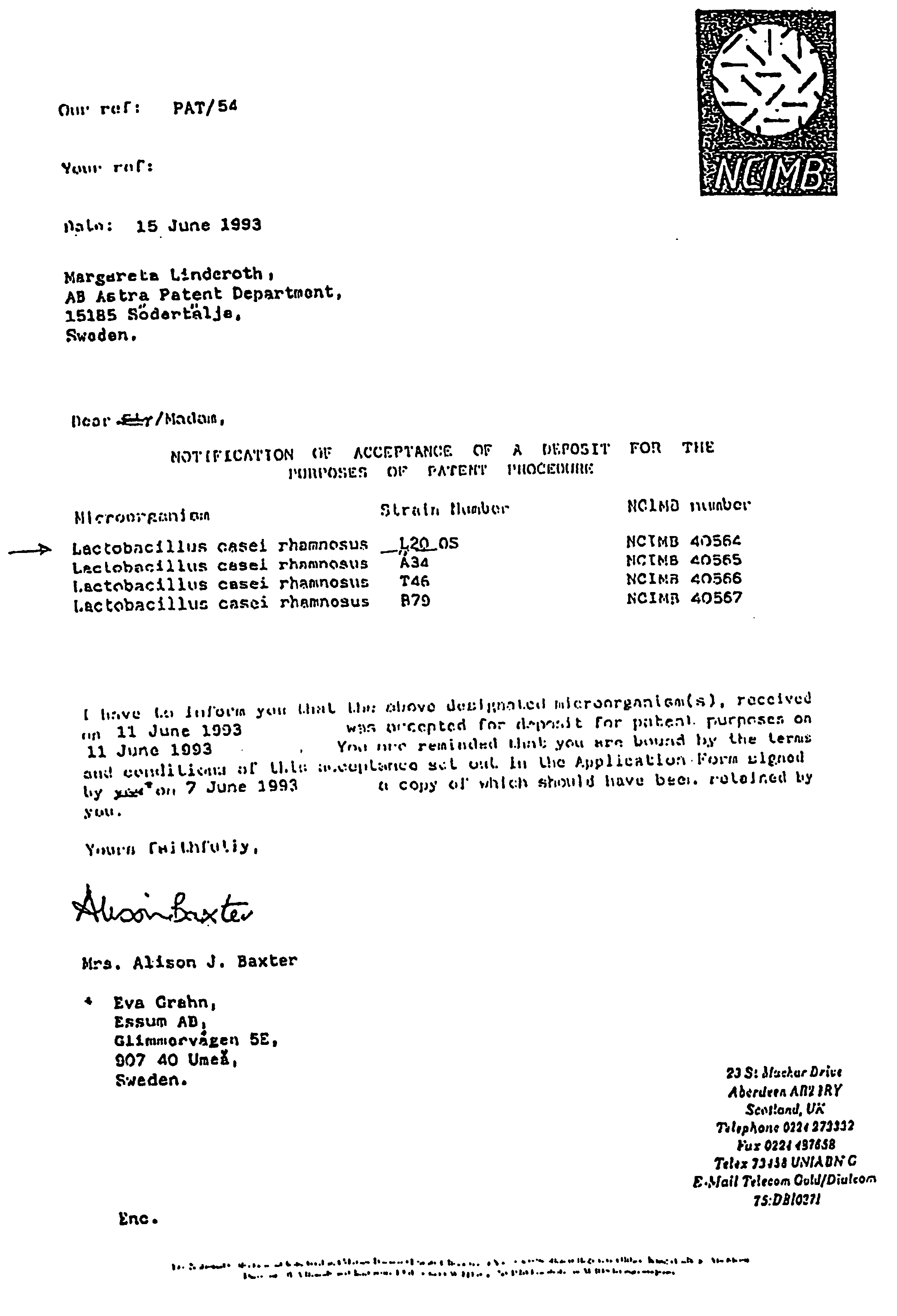
Our ref: PAT/54

Your ref:

Date: 15 June 1993

Margareta Linderoth,
AB Astra Patent Department,
15185 Södertälje,
Sweden.

NCIMB

Dear Sir/Madam,

NOTIFICATION OF ACCEPTANCE OF A DEPOSIT FOR THE
PURPOSES OF PATENT PROCEDURE

Microorganism | Strain Number | NCIMB number
Lactobacillus casei rhamnosus | L20 05 | NCIMB 40564
Lactobacillus casei rhamnosus | A34 | NCIMB 40565
Lactobacillus casei rhamnosus | T46 | NCIMB 40566
Lactobacillus casei rhamnosus | B79 | NCIMB 40567

I have to inform you that the above designated microorganism(s), received
on 11 June 1993 was accepted for deposit for patent purposes on
11 June 1993 . You are reminded that you are bound by the terms
and conditions of this acceptance set out in the Application Form signed
by you* on 7 June 1993 a copy of which should have been retained by
you.

Yours faithfully,


Mrs. Alison J. Baxter

* Eva Grahn,
Essum AB,
Glimmervägen 5E,
907 40 Umeå,
Sweden.

23 St Machar Drive
Aberdeen AB2 1RY
Scotland, UK
Telephone 0224 273332
Fax 0224 487658
Telex 73458 UNIABN G
E-Mail Telecom Gold/Dialcom
75:DBI0271

Enc.

[?]
[?]

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

[?]va Grahn,
[?]ssum AB,
[?]limmervågen 5E,
[?]07 40 Umeå,
Sweden

VIABILITY STATEMENT
issued pursuant to Rule 10.2 by the
INTERNATIONAL DEPOSITARY AUTHORITY
identified on the following page

NAME AND ADDRESS OF THE PARTY
TO WHOM THE VIABILITY STATEMENT
IS ISSUED

DEPOSITOR | II. IDENTIFICATION OF THE MICROORGANISM
Name: | Accession number given by the
AS ABOVE | INTERNATIONAL DEPOSITARY AUTHORITY:
Address: | NCIMB 40564
| Date of the deposit or of the transfer:
| 11 June 1993

III. VIABILITY STATEMENT
The viability of the microorganism identified under II above was tested
on 14 June 1993 2. On that date, the said microorganism was
X 3 viable
3 no longer viable

Indicate the date of the original deposit or, where a new deposit or a transfer has been
made, the most recent relevant date (date of the new deposit or date of the transfer).
2 In the cases referred to in Rule 10.2(a)(ii) and (iii), refer to the most recent viability
test.
3 Mark with a cross the applicable box.

Form BP/9 (first page)

IV. CONDITIONS UNDER WHICH THE VIABILITY TEST HAS BEEN PERFORMED⁴

INTERNATIONAL DEPOSITARY AUTHORITY

Name: [?][?]IMB Ltd | Signature(s) of person(s) having the power
23 St Machar Drive | to represent the International Depositary
Address: Aberdeen Scotland | Authority or of authorized official(s):
UK AB2 1RY | [signature]
| Date: 15 June 1993

⁴ Fill in if the information has been requested and if the results of the test were negative.

Form BP/9 (second and last page)

## Claims

1. A pharmaceutical preparation for prophylaxis against and/or treatment of gastrointestinal disorders in man and animals, **characterised in that** it comprises a viable microorganism strain in the form of the Lactobacillus casei rhamnosus strains LB21 with the accession number NCIMB 40564 in at least one pharmaceutically acceptable carrier medium, in which the microorganism maintains its viability.

2. A pharmaceutical preparation as claimed in claim 1, **characterised in that** the microorganism strain is present in an amount of 10²-10¹² colony-forming units per ml of ready-to-use preparation.

3. A pharmaceutical preparation as claimed in claim 2, **characterised in that** the microorganism strain is present in an amount of 10⁶-10¹⁰ colony-forming units per ml of ready-to-use preparation.

4. A pharmaceutical preparation as claimed in claim 1, **characterised in that** the pharmaceutical acceptable carrier medium is a soured or fermented milk product, preferably sour milk, yoghurt and milk or fruit juice, ice-cream, soup and fruit drinks.

5. A pharmaceutical preparation as claimed in any one of the preceding claims, **characterised in that** the pharmaceutical acceptable carrier medium is a suitable animal feedstuff, preferably whey, dry fodder or a concentrated suspension, when administering the preparation according to the invention to animals.

6. A pharmaceutical preparation as claimed in claim 1, **characterised in that** the bacterial strain has been added to the carrier medium as a starting culture and/or to the ready-to-use preparation.

7. A pharmaceutical preparation as claimed in any one of the preceding claims, **characterised in that** it is present in dry form, such as capsules, tablets or powder, or in the form of a suspension, optionally frozen, preferably NaCl solution, glucose solution or skim milk, together with a pharmaceutically acceptable carrier material.

8. A pharmaceutical preparation as claimed in any one of the preceding claims, **characterised in that** at least one other Lactococcus, Lactobacillus or Bifidobacterium strain, preferably Lactococcus lactis L1a, is also incorporated into the preparation.

9. A Lactobacillus casei rhamnosus strain capable of preventing and/or curing gastrointestinal disorders in man and animals, wherein it has the accession No. NCIMB 40564.

10. Use of a Lactobacillus casei rhamnosus strain according to claim 9 for the production of a medicament for prophylaxis against and/or treatment of antibiotic-associated diarrhoea, helicobacter infection in the stomach and in the upper small intestine, tourist diarrhoea, stress-induced gastrointestinal disorders, cytotoxic-induced intestinal influence, allergies in children, constipation and/or for prophylaxis against intestinal infections in children having undergone a liver transplant or been operated for bile duct atresia or in babies that are newborn or prematurely born.

11. Process for the production of a pharmaceutical preparation according to any one of claims 1-8, wherein the Lactobacillus casei rhamnosus strain LB21 with the accessionnumber NCIMB 40564 isolated from faeces of a 1-8 day-old baby, preferably 5 days old, is added to a pharmaceutically acceptable carrier medium, in which the microorganism maintains its viability.

## Patentansprüche

1. Pharmazeutische Zubereitung zur Prophylaxe gegen und/oder Behandlung von gastrointestinalen Störungen im Menschen und in Tieren, **dadurch gekennzeichnet, dass** sie einen lebensfähigen Mikroorganismus-Stamm in Form der Lactobacillus casei rhamnosus-Stämme LB21 mit der Zugangs-Nr. NCIMB 40564 in wenigstens einem pharmazeutisch annehmbaren Träger-Medium umfasst, in dem der Mikroorganismus seine Lebensfähigkeit beibehält.

2. Pharmazeutische Zubereitung nach Anspruch 1, **dadurch gekennzeichnet, dass** der Mikroorganismus-Stamm in einer Menge von 10² bis 10¹² Koloniebildenden Einheiten pro ml der für eine Verwendung fertigen Zubereitung zugegen ist.

3. Pharmazeutische Zubereitung nach Anspruch 2, **dadurch gekennzeichnet, dass** der Mikroorganismus-Stamm in einer Menge von 10⁶ bis 10¹⁰ Koloniebildenden Einheiten pro ml der für eine Verwendung fertigen Zubereitung zugegen ist.

4. Pharmazeutische Zubereitung nach Anspruch 1, **dadurch gekennzeichnet, dass** das pharmazeutisch annehmbare Träger-Medium ein gesäuertes oder fermentiertes Milch-Produkt ist, vorzugsweise Sauermilch, Joghurt und Milch, oder Fruchtsaft, Eiscreme, Suppe und Frucht-Getränke ist.

5. Pharmazeutische Zubereitung nach einem der vorangehenden Ansprüche, **dadurch gekennzeichnet, dass** das pharmazeutisch annehmbare Träger-Medium ein geeignetes Tierfutter, vorzugsweise Molke, Trockenfutter oder eine konzentrierte Suspension ist, wenn die Zubereitung gemäß der Erfindung an Tiere verabreicht wird.

6. Pharmazeutische Zubereitung nach Anspruch 1, **dadurch gekennzeichnet, dass** der Bakterienstamm dem Träger-Medium als Start-Kultur zugesetzt wurde und/oder der für die Verwendung fertigen Zubereitung zugesetzt wurde.

7. Pharmazeutische Zubereitung nach einem der vorangehenden Ansprüche, **dadurch gekennzeichnet, dass** sie in trockener Form wie beispielsweise in Form von Kapseln, Tabletten oder Pulver, oder in Form einer Suspension, gegebenenfalls gefroren, vorzugsweise als NaCl-Lösung, Glucose-Lösung oder als Magermilch, zusammen mit einem pharmazeutisch annehmbaren Träger-Material vorliegt.

8. Pharmazeutische Zubereitung nach einem der vorangehenden Ansprüche, **dadurch gekennzeichnet, dass** wenigstens ein anderer Lactococcus-, Lactobacillus- oder Bifidobacterium-Stamm, vorzugsweise Lactococcus lactis L1a, ebenfalls in die Zubereitung eingearbeitet ist.

9. Lactobacillus casei rhamnosus-Stamm, der in der Lage ist, gastrointestinale Störungen im Menschen und in Tieren vorzubeugen und/oder diese zu heilen, worin der Stamm die Zugangs-Nr. NCIMB 40564 aufweist.

10. Verwendung eines Lactobacillus casei rhamnosus-Stamms gemäß Anspruch 9 für die Herstellung eines Medikaments zur Prophylaxe gegen und/oder Behandlung von Antibiotika-assoziierter Diarrhoe, Heliobacter-Infektion im Magen und im oberen Dünndarm, Touristen-Diarrhoe, Stress-induzierter gastrointestinaler Störungen, Cytotoxin-induzierter Einflüsse auf den Darm, Allergien bei Kindern, Verstopfung und/oder zur Prophylaxe gegen Darm-Infektionen bei Kindern, die sich einer Leber-Transplantation unterzogen haben oder wegen einer Gallengang-Atresie operiert wurden, oder bei Babys, die frisch geboren oder vorzeitig geboren wurden.

11. Verfahren zur Herstellung einer pharmazeutischen Zubereitung nach einem der Ansprüche 1 bis 8, worin der Lactobacillus casei rhamnosus-Stamm LB21 mit der Zugangs-Nr. NCIMB 40564, der von Faeces eines ein bis acht Tage alten Babys isoliert wurde, vorzugsweise eines fünf Tage alten Babys isoliert wurde, einem pharmazeutisch annehmbaren Träger-Medium zugesetzt wird, in dem der Mikroorganismus seine Lebensfähigkeit beibehält.

## Revendications

1. Préparation pharmaceutique pour la prophylaxie contre et/ou le traitement des troubles gastro-intestinaux chez l'homme et l'animal, **caractérisée en ce qu'**elle comprend une souche de micro-organisme viable sous la forme des souches LB21 de Lactobacillus casei rhamnosus ayant le numéro d'accès NCIMB 40564 dans au moins un excipient pharmaceutiquement acceptable, dans lequel le micro-organisme conserve sa viabilité.

2. Préparation pharmaceutique selon la revendication 1, **caractérisée en ce que** la souche du micro-organisme est présente en une quantité de 10²-10¹² unités formant colonie par ml de préparation prête à l'emploi.

3. Préparation pharmaceutique selon la revendication 2, **caractérisée en ce que** la souche du micro-organisme est présente en une quantité de 10⁶-10¹⁰ unités formant colonie par ml de préparation prête à l'emploi.

4. Préparation pharmaceutique selon la revendication 1, **caractérisée en ce que** l'excipient pharmaceutique acceptable est un produit laitier ayant tourné ou fermenté, de préférence, le lait aigre, le yaourt et le lait ou un jus de fruit, une crème glacée, la soupe ou des boissons aux fruits.

5. Préparation pharmaceutique selon l'une quelconque des revendications précédentes, **caractérisée en ce que** l'excipient pharmaceutique acceptable est un aliment pour animaux convenable, de préférence, le petit-lait, le fourrage sec ou une suspension concentrée, lorsque la préparation selon l'invention est administrée à des animaux.

6. Préparation pharmaceutique selon la revendication 1, **caractérisée en ce que** la souche bactérienne a été ajoutée à l'excipient à titre de culture de départ et/ou à la préparation prête à l'emploi.

7. Préparation pharmaceutique selon l'une quelconque des revendications précédentes, **caractérisée en ce qu'**elle est présente sous une forme sèche, telle que des gélules, des comprimés ou une poudre, ou sous la forme d'une suspension, éventuellement congelée, de préférence, une solution de NaCl, une solution de glucose ou du lait écrémé, conjointement avec un matériau de type excipient pharmaceutiquement acceptable.

8. Préparation pharmaceutique selon l'une quelconque des revendications précédentes, **caractérisée en ce qu'**au moins une autre souche de Lactococcus, Lactobacillus ou Bifidobacterium, de préférence, Lactococcus lactis L1a, est également incorporée dans la préparation.

9. Souche de Lactobacillus casei rhamnosus capable de prévenir et/ou de guérir les troubles gastro-intestinaux chez l'homme et l'animal, ayant le N° d'accès NCIMB 40564.

10. Utilisation d'une souche de Lactobacillus casei rhamnosus selon la revendication 9 pour la fabrication d'un médicament pour la prophylaxie contre et/ou le traitement des diarrhées associées aux antibiotiques, une infection par Helicobacter de l'estomac ou de l'intestin grêle supérieur, la diarrhée des touristes, les troubles gastro-intestinaux induits par le stress, l'influence intestinale de type cytotoxique, les allergies chez l'enfant, la constipation, et/ou pour la prophylaxie contre les infections intestinales chez les enfants ayant subi une greffe de foie ou qui ont été opérés d'une atrésie des voies biliaires ou chez les bébés nouveaux-nés ou prématurés.

11. Procédé de production d'une préparation pharmaceutique selon l'une quelconque des revendications 1 à 8, dans lequel la souche LB21 de Lactobacillus casei rhamnosus ayant le numéro d'accès NCIMB 40564 isolée à partir des selles d'un bébé de 1 à 8 jours, de préférence, de 5 jours, est ajoutée à un excipient pharmaceutiquement acceptable, dans lequel le micro-organisme conserve sa viabilité.
